# EUROPEAN PATENT APPLICATION

(11) **EP 2 505 196 A1**
(43) Date of publication of application: **03.10.2012**
(21) Application number: 10833290.9
(22) Date of filing: 25.11.2010
(51) Int. Cl.: A61K 31/185, A61P 7/02, A61P 9/00, A61P 9/10, A61P 11/00, A61P 25/00

(54) **ANTITHROMBOTIC AGENT**

(30) Priority: 27.11.2009 JP 2009270185; 30.03.2010 JP 2010077899
(71) Applicant: Tokai University Educational System, Tokyo 151-0063 (JP); Taiho Pharmaceutical Co., Ltd., Chiyoda-ku, Tokyo 1018444 (JP)
(72) Inventor: ISHIDA, Hideyuki, Isehara-shi Kanagawa 259-1193 (JP); KINIWA, Mamoru, Tokushima-shi Tokushima 771-0194 (JP)
(74) Representative: Harmsen, Dirk
(86) International application number: PCT/JP2010/071058
(87) International publication number: WO 2011/065444

(57) **Abstract**

An object of the present invention is to provide an antithrombotic agent that has fewer side effects and that is highly effective. The present invention provides an antithrombotic agent comprising (±)-[2-[4-(3-ethoxy-2-hydroxypropoxy)phenylcarbamoyl]ethyl]dimethylsulfonium p-toluenesulfonate represented by Formula (1) as an active ingredient.

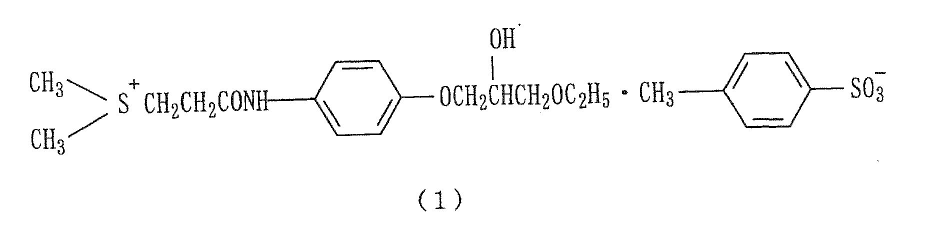

## Description

### Technical Field

The present invention relates to an antithrombotic agent comprising (±)-[2-[4-(3-ethoxy-2-hydroxypropoxy)phenylcarbamoyl]ethyl]dimethylsulfonium p-toluenesulfonate as an active ingredient.

### Background Art

Vascular disease (thrombosis), which is a frequent cause of heart disease and encephalopathy, suddenly causes serious diseases such as myocardial infarction and brain infarction, resulting in death in not a few cases. In addition, it is also known that the onset of secondary thrombosis, such as disseminated intravascular coagulation in cancer and leukemia, deep vein thrombosis after various surgical operations, and subsequent pulmonary embolism, leads to death in some cases. Furthermore, many diabetics shift to hemodialysis due to peripheral circulatory disturbance or renal failure. Thus, also in light of prevention of blood coagulation in such a blood extracorporeal circulation path, the search for antithrombotic agents is still a social issue.

As therapeutic agents for thrombosis, thrombolytic agents such as tissue plasminogen activator (t-PA), platelet aggregation inhibitors such as tirofiban and aspirin, anticoagulants such as heparin and warfarin, and the like are used. However, warfarin, for example, may cause serious side effects including cerebral hemorrhage. Hence, there is a need for safer and more effective antithrombotic agents.

On the other hand, (±)-[2-[4-(3-ethoxy-2-hydroxypropoxy)phenylcarbamoyl]ethyl]dimethylsulfonium p-toluenesulfonate (hereinafter also referred to as "suplatast tosilate"), which has excellent suppressing action against IgE antibody production, is known as a therapeutic agent for bronchial asthma, atopic dermatitis, and allergic rhinitis (Patent Literature 1). Suplatast tosilate is also known to be useful as a therapeutic agent for urination disorder (Patent Literature 2), a therapeutic agent for itch associated with kidney dialysis (Patent Literature 3), an agent for ameliorating liver dysfunction caused by C-type or non-B, non-C hepatitis virus (Patent Literature 4), and a therapeutic agent for chemical sensitivity (Patent Literature 5). However, it has been completely unknown that suplatast tosilate has an excellent effect as an antithrombotic agent.

### Citation List

### Patent Literature

PTL 1: Japanese Examined Patent Publication No. H3-70698
PTL 2: WO00/27383
PTL 3: Japanese Unexamined Patent Publication No. H11-315019
PTL 4: Japanese Unexamined Patent Publication No. 2002-114672
PTL 5: Japanese Unexamined Patent Publication No. 2004-292407

### Summary of Invention

### Technical Problem

An object of the present invention is to provide an antithrombotic agent that has fewer side effects and that is highly effective.

### Solution to Problem

The present inventors conducted extensive research on compounds useful in the treatment of thrombosis, and found that suplatast tosilate has excellent antithrombotic action.

Specifically, the present invention relates to the following inventions.
1. An antithrombotic agent comprising (±)-[2-[4-(3-ethoxy-2-hydroxypropoxy)phenylcarbamoyl]ethyl]dimethylsulfonium p-toluenesulfonate represented by Formula (1) as an active ingredient.

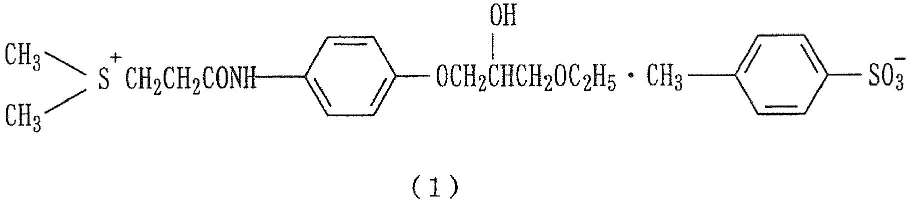

2. The antithrombotic agent according to 1 which is effective in the prevention or treatment of thrombosis selected from the group consisting of myocardial infarction, brain infarction, pulmonary infarction, arteriosclerosis obliterans, Buerger disease, thrombophlebitis, venous thromboembolism (economy class syndrome), arterial thromboembolism, and disseminated intravascular coagulation (DIC).
3. An antithrombotic agent comprising a combination of (±)-[2-[4-(3-ethoxy-2-hydroxypropoxy)phenylcarbamoyl]ethyl]dimethylsulfonium p-toluenesulfonate represented by Formula (1) and a platelet aggregation inhibitor.

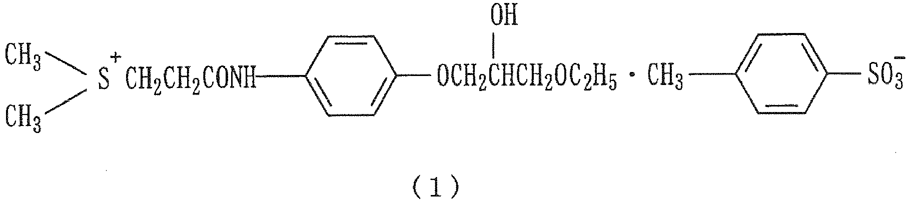

4. The antithrombotic agent according to 3 which is in a pharmaceutical form comprising (±)-[2-[4-(3-ethoxy-2-hydroxypropoxy)phenylcarbamoyl]ethyl]dimethylsulfonium p-toluenesulfonate represented by Formula (1) and a platelet aggregation inhibitor.
5. The antithrombotic agent according to 3 or 4 which is a kit comprising (±)-[2-[4-(3-ethoxy-2-hydroxypropoxy)phenylcarbamoyl]ethyl]dimethylsulfonium p-toluenesulfonate and a platelet aggregation inhibitor.
6. The antithrombotic agent according to any one of 3 to 5, wherein the platelet aggregation inhibitor is cilostazol.
7. A method for treating thrombosis, comprising administering to a patient in need of such treatment an effective amount of (±)-[2-[4-(3-ethoxy-2-hydroxypropoxy)phenylcarbamoyl]ethyl]dimethylsulfonium p-toluenesuifonate represented by Formula (1).

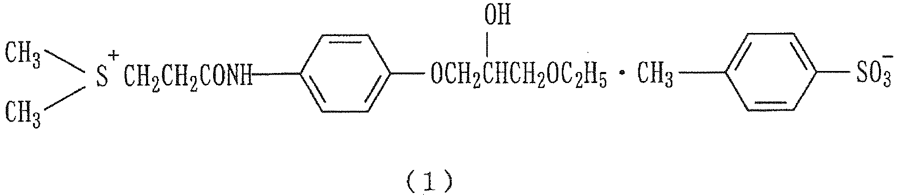

8. The method according to 7, wherein the thrombosis is selected from the group consisting of myocardial infarction, brain infarction, pulmonary infarction, arteriosclerosis obliterans, Baerger disease, thrombophlebitis, venous thromboembolism (economy class syndrome), arterial thromboembolism, and disseminated intravascular coagulation (DIC).
9. (±)-[2-[4-(3-Ethoxy-2-hydroxypropoxy)phenylcarbamoyl]ethyl]dimethylsulfonium p-toluenesulfonate represented by Formula (1) for use in the treatment of thrombosis.

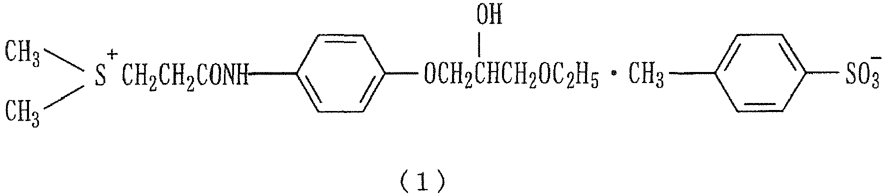

10. Use of (±)-[2-[4-(3-ethoxy-2-hydroxypropoxy)phenylcarbamoyl]ethyl]dimethylsulfonium p-toluenesulfonate represented by Formula (1) for the treatment of thrombosis.

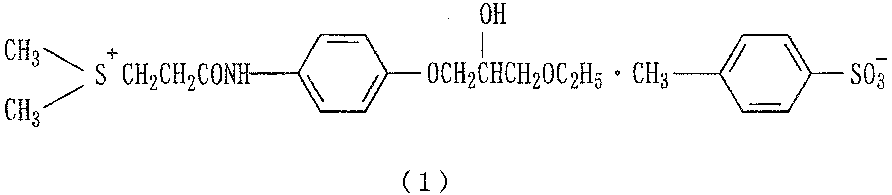

### Advantageous Effects of Invention

The therapeutic agent of the present invention exhibits an excellent effect in the treatment of vascular disease, in particular, thrombosis, and is very useful with fewer side effects.

### Brief Description of Drawings

Fig. 1 shows the results of thrombus formation inhibitory action in Test Example 1.
Fig. 2 shows the results of the amount of bleeding (side effect) in Test Example 2. A: Single-dose administration of suplatast tosilate B: Administration of suplatast tosilate for 7 consecutive days
Fig. 3 shows the results of the effect of inhibiting thrombus formation of combined use with cilostazol in Test Example 3.

### Description of Embodiments

(+)-[2-[4-(3-Ethoxy-2-hydroxypropoxy)phenylcarbamoyl]ethyl]dimethylsulfonium p-toluenesulfonate (suplatast tosilate), an active ingredient of the present invention, is a known compound and can be produced by the method described, for example, in Japanese Examined Patent Publication H3-70698.

"Treatment" as used in the present invention means maintenance therapy for prevention and treatment of diseases, as well as amelioration of symptoms and recurrence prevention.

Vascular disease against which suplatast tosilate is effective, in particular, thrombosis, refers to diseases, such as myocardial infarction, brain infarction, pulmonary infarction, arteriosclerosis obliterans, Buerger disease, thrombophlebitis, venous thromboembolism (economy class syndrome), arterial thromboembolism, and disseminated intravascular coagulation (DIC).

Suplatast tosilate can be used in combination with other drugs for thrombosis in the prevention or treatment of thrombosis.

In the present invention, examples of drugs that can be used in combination with suplatast tosilate include thrombolytic agents, platelet aggregation inhibitors, anticoagulants, etc. Combined use of suplatast tosilate and one or more of these drugs not only further improves the therapeutic effect of the antithrombotic agent of the compound of the present invention, but also enables the dose to be reduced, as compared to the case where each of the drugs that can be used in combination is used alone; thus, it is also suitable in terms of reducing side effects. In addition, stent treatment, etc., may be used in combination.

Examples of thrombolytic agents that can be used in combination with suplatast tosilate include urokinase (u-PA), streptokinase, tissue plasminogen activator (t-PA), etc.

Examples of platelet aggregation inhibitors that can be used in combination with suplatast tosilate include tirofiban, aspirin, cilostazol, etc.

Examples of anticoagulants that can be used in combination with suplatast tosilate include heparin, warfarin, etc.

When suplatast tosilate may form hydrates, they are also within the scope of the present invention.

The antithrombotic agent of the present invention can be administered in various forms to humans. Such forms include, for example, oral preparations, injections, rectal suppositories, and external preparations (such as ointments, patches, and eye drops). These preparations can be produced by common methods well known by a person skilled in the art.

Among oral preparations, solid preparations can be prepared as follows. Excipients, optionally together with binders, disintegrants, lubricants, colorants, sweetening agents, flavoring agents, etc., are added into suplatast tosilate and then processed using an ordinary method into tablets, coated tablets, granules, powders, capsules, dry syrups, or the like. Liquid oral preparations can be prepared as follows. Sweetening agents, buffers, stabilizers, flavoring agents, etc., are added into suplatast tosilate and processed using an ordinary method into internal liquid medicines, syrups, or the like.

Injections can be prepared as follows. pH adjusting agents, buffers, stabilizers, tonicity adjusting agents, topical anesthetics, etc., are added into suplatast tosilate and processed using an ordinary method into subcutaneous injections, intramuscular injections, intravenous injections, or the like.

Suppositories for rectal administration can be prepared by adding an excipient into suplatast tosilate, optionally together with a surfactant, etc., followed by a process using an ordinary method.

Among external preparations, ointments, such as in the form of paste, cream, or gel, can be prepared as follows. Stabilizers, wetting agents, preservatives, etc., are added as required into a base containing suplatast tosilate, and processed and formulated using an ordinary method. Examples of the base include white petrolatum, paraffin, glycerin, cellulose derivatives, polyethylene glycol, silicone, bentonite, etc. Examples of the preservative include methyl parahydroxybenzoate, ethyl parahydroxybenzoate, propyl parahydroxybenzoate, etc.

In addition, patches as external preparations can be produced by applying the above ointment, cream, gel, paste, etc., onto an ordinary support, using an ordinary method. Preferable examples of supports include woven or nonwoven fabrics made from cotton, staple fibers, and chemical fibers; and films and foam sheets of soft vinyl chloride, polyethylene, polyurethane, etc.

The drugs that can be used in combination with suplatast tosilate can also be presented in the same dosage form as that of suplatast tosilate mentioned above.

Therefore, when two or more ingredients, i.e., suplatast tosilate and one or more drugs that can be used in combination with suplatast tosilate, are used, the antithrombotic agent of the present invention may take a single dosage form containing these ingredients, or two or more dosage forms containing the ingredients individually. In the case of two or more dosage forms, it may be provided as a kit in the forms taken via the same administration route (for example, both are oral preparations), or as a kit in the forms taken via different administration routes (for example, one is an oral preparation and the other is an injection). Moreover, as long as the object of the present invention is achieved, separately prepared preparations may be administered simultaneously or at different times to the same subject, and the dose frequency per day of each preparation may be different.

The amount of suplatast tosilate to be contained in each of the preparations mentioned above can be determined as appropriate depending on the patient's condition, dosage form, etc. In general, the desirable amount is about 5 to 10,000 mg (preferably 5 to 1,000 mg) in the case of an oral preparation, about 0.1 to 5,000 mg (preferably 0.1 to 500 mg) in the case of an injection, and about 5 to 10,000 mg (preferably 5 to 1,000 mg) in the case of a suppository and an external preparation, per dosage unit for an adult.

Further, the daily dose of suplatast tosilate may also vary depending on the patient's condition, body weight, age, sex or other conditions. The desirable daily dose for an adult is about 0.1 to 10,000 mg (preferably 5 to 1,000 mg). The preparations can be administered in a single dose, or two to four divided doses per day.

In a preferred embodiment of the present invention, suplatast tosilate and a platelet aggregation inhibitor are administered in combination. The doses of the suplatast tosilate and the platelet aggregation inhibitor are not particularly limited, as long as antithrombotic effect is potentiated. For example, the daily dose of the platelet aggregation inhibitor may be about 1 to 5,000 mg, preferably about 5 to 1,000 mg, per 300 mg of the suplatast tosilate. Suplatast tosilate and platelet aggregation inhibitors, in particular, cilostazol, exhibit potent synergistic effect with respect to antithrombotic effect; therefore, although the daily dose of each drug may be administered, the dose of each drug can be reduced to, for example, about 50 to 95% of the effective amount of each drug used alone to reduce the side effects of each drug.

In a preferred embodiment, the antithrombotic agent of the present invention may be formulated into one dosage form (one dosage form preparation) as a combination drug (preparation containing a plurality of active ingredients) that comprises suplatast tosilate and a platelet aggregation inhibitor at the above ratio; or may be formulated into a plurality of dosage forms (multiple dosage form preparation) by formulating the above active ingredients as single drugs (preparations containing a single active ingredient) to be used simultaneously, or at separate intervals.

Regarding the antithrombotic agent of the present invention, as long as suplatast tosilate and a platelet aggregation inhibitor are administered in combination, each of the preparations mentioned above may be individually produced, packed, and distributed; or all or a part of the preparations may be produced, packed, and distributed as a single package (kit formulation) suitable for administering in combination.

When the antithrombotic agent of the present invention in which suplatast tosilate is used in combination with a platelet aggregation inhibitor is provided as a kit, individual preparations may be administered simultaneously, or at separate intervals. In the present invention, the suplatast tosilate and the platelet aggregation inhibitor can be packaged in a kit comprising a combination of pharmaceutical compositions for the treatment of thrombosis in a mammal, comprising:
(a) an antithrombotic composition containing a therapeutically effective amount of suplatast tosilate, and
(b) an antithrombotic composition containing a therapeutically effective amount of a platelet aggregation inhibitor.
The compositions constituting the kit may be in any known pharmaceutical form, and usually placed in any commonly used containers according to their pharmaceutical form.

### Examples

Test Examples are given below to illustrate the present invention in more detail; however, the present invention is not limited to these Examples.

### Test Example 1

### Thrombus Formation Inhibitory Action

Male ICR mice (ten weeks old) were used. Rhodamine 6G (platelet fluorescence-labeling agent) was administered to the tail vein under anesthesia. With the abdomen open, the arteria testicularis was subjected to FeCl₃ treatment to induce thrombus formation. Thrombus formation was observed by a real time color three-dimensional observation device, and the thrombus formation time was measured. Suplatast tosilate (IPD), the compound of the present invention, was dissolved in water and orally administered in a single dose (100 mg/kg) 30 minutes before the FeCl₃ treatment or for 7 consecutive days (100 mg/kg, 30 mg/kg, 10 mg/kg) before the FeCl₃ treatment. In addition, as a control drug, tirofiban (0.3 µmol/kg) was intravenously administered.

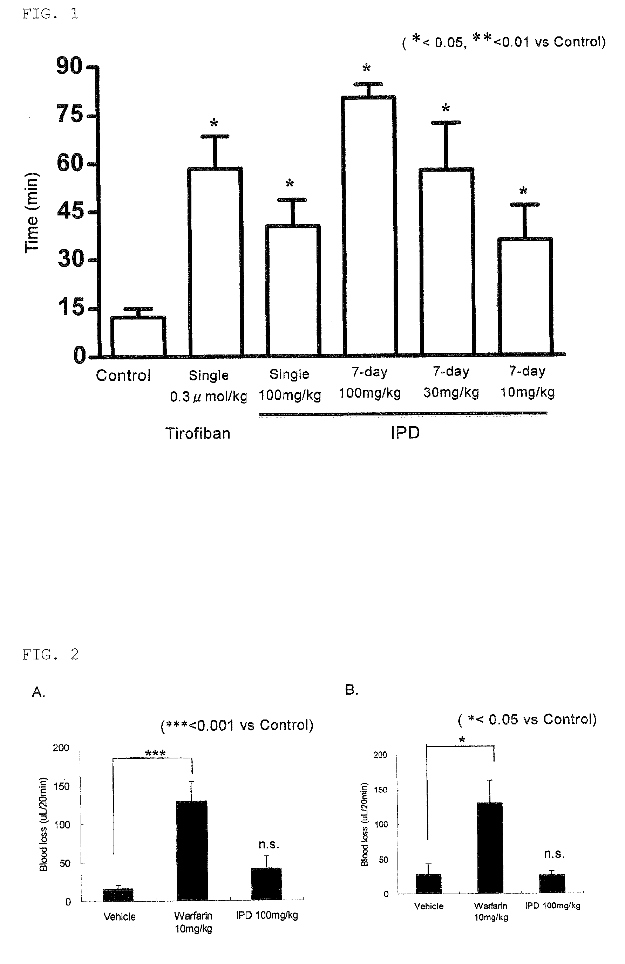

As shown in Fig. 1, the results reveal that in the control group (group that was given water), a large thrombus was formed about 14 minutes after the FeCl₃ stimulation. On the other hand, significant prolongation of large thrombus formation time was observed as 60 minutes in the group that was intravenously given tirofiban (0.3 µmol/kg), a potent platelet aggregation inhibitor, and as 45 minutes in the group that was orally given IPD (100 mg/kg) in a single dose.

Moreover, large thrombus formation inhibitory action of IPD was clearly enhanced by the oral administration for 7 consecutive days, and large thrombus formation inhibitory action of the 7-day administration at 10 mg/kg/day was similar to that of the oral administration in a single dose of 100 mg/kg.

### Test Example 2

Male ICR mice (ten weeks old) were used. The last 2 mm of the tail was amputated with a scalpel under pentobarbital anesthesia, and the tail 2 cm from the tip was immediately immersed in 10 mL of physiological saline at 37°C. Twenty minutes later, the tail was removed from the physiological saline, and 1 mL of Triton X-100 (25% in water) was added. The absorbance at 546 nm was measured, and the amount of bleeding was determined from a calibration curve. Suplatast tosilate (IPD, 100 mg/kg), the compound of the present invention, and a control drug, warfarin (10 mg/kg) were dissolved in distilled water and orally administered in a single dose one hour before amputating the tail, or orally administered for 7 consecutive days before amputating the tail. Distilled water was administered to the control.

As shown in Fig. 2, the results reveal that 10 mg/kg of warfarin, an anticoagulant, exhibited the notable effect of increasing the amount of bleeding by the single-dose administration and consecutive administration, whereas IPD (100 mg/kg) did not have a significant effect on the amount of bleeding in either the single-dose administration or administration for 7 consecutive days.

### Test Example 3

Male ICR mice (ten weeks old) were used. Rhodamine 6G (platelet fluorescence-labeling agent) was administered to the tail vein under anesthesia. With the abdomen open, the arteria testicularis was subjected to FeCl₃ treatment to induce thrombus formation. Thrombus formation was observed by a real time color three-dimensional observation device, and thrombus formation time was measured. Suplatast tosilate (IPD) (10 mg/kg) was dissolved in water and orally administered for 7 days before amputating the tail to the group that was given IPD alone. Cilostazol (10 mg/kg, 30 mg/kg, 100 mg/kg), a platelet aggregation inhibitor, was dissolved in water and orally administered in a single dose one hour before amputating the tail to the group that was given cilostazol alone. To the combination group of IPD and cilostazol, IPD (10 mg/kg) was orally administered for 7 days, and cilostazol (10 mg/kg) was orally administered in a single dose one hour before amputating the tail on the 7th day of the IPD administration.

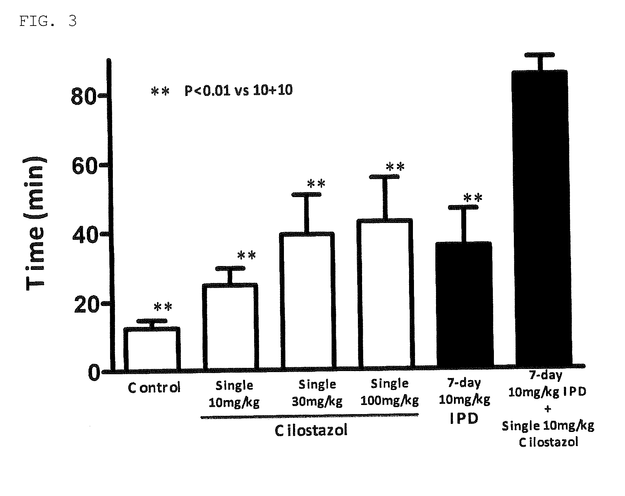

Fig. 3 shows the results. Due to the FeCl₃ treatment, thrombus formation was observed in the arteria testicularis, and thrombi were becoming larger with time. In the control group (group that was given water), large thrombus formation was observed 12±3 minutes after the FeCl₃ treatment. Both in the group that was given IPD alone and in the group that was given cilostazol alone, the thrombus formation time was significantly prolonged, i.e., 36±11 minutes in the group that was given IPD alone, and 25±5 minutes in the group that was given cilostazol alone (P<0.05 vs. cont.); and this action was dose-dependent.

The thrombus formation time in the combination group of IPD and cilostazol was notably prolonged as 85±5 minutes (P<0.01 vs. cont.), and was significant even compared to the group that was given IPD alone and the group that was given cilostazol alone (P<0.01 vs. IPD, P<0.01 vs. cilostazol; one-way ANOVA, Newman-Keuls Multiple Comparison Test).

The above-described results reveal that suplatast tosilate, the compound of the present invention, has thrombus formation inhibitory action, and that the action is further enhanced by consecutive administration. Additionally, it was revealed that unlike anticoagulants, this action of suplatast tosilate is not accompanied by effects of increasing the amount of bleeding and prolonging bleeding time.

Furthermore, it was revealed that the thrombus formation inhibitory action is synergistically enhanced by combined use with a platelet aggregation inhibitor (cilostazol).

## Claims

1. An antithrombotic agent comprising (±)-[2-[4-(3-ethoxy-2-hydroxypropoxy)phenylcarbamoyl]ethyl]dimethylsulfonium p-toluenesulfonate represented by Formula (1) as an active ingredient.

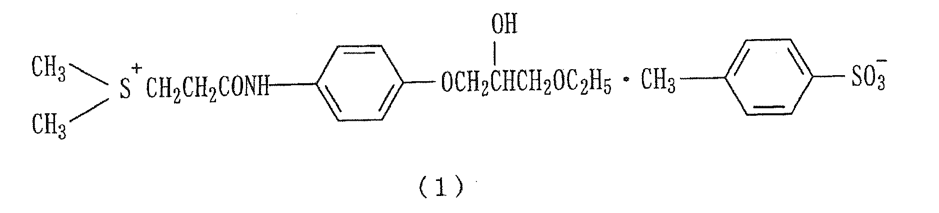

2. The antithrombotic agent according to Claim 1 which is effective in the prevention or treatment of thrombosis selected from the group consisting of myocardial infarction, brain infarction, pulmonary infarction, arteriosclerosis obliterans, Buerger disease, thrombophlebitis, venous thromboembolism (economy class syndrome), arterial thromboembolism, and disseminated intravascular coagulation (DIC).

3. An antithrombotic agent comprising a combination of (±)-[2-[4-(3-ethoxy-2-hydroxypropoxy)phenylcarbamoyl]ethyl]dimethylsulfonium p-toluenesulfonate represented by Formula (1) and a platelet aggregation inhibitor.

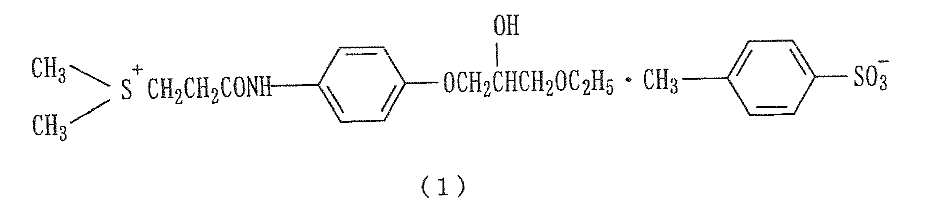

4. The antithrombotic agent according to Claim 3 which is in a pharmaceutical form comprising (±)-[2-[4-(3-ethoxy-2-hydroxypropoxy)phenylcarbamoyl]ethyl]dimethylsulfonium p-toluenesulfonate represented by Formula (1) and a platelet aggregation inhibitor.

5. The antithrombotic agent according to Claim 3 which is a kit comprising (±)-[2-[4-(3-ethoxy-2-hydroxypropoxy)phenylcarbamoyl]ethyl]dimethylsulfonium p-toluenesulfonate and a platelet aggregation inhibitor.

6. The antithrombotic agent according to Claim 3, wherein the platelet aggregation inhibitor is cilostazol.

7. A method for treating thrombosis, comprising administering to a patient in need of such treatment an effective amount of (±)-[2-[4-(3-ethoxy-2-hydroxypropoxy)phenylcarbamoyl]ethyl]dimethylsulfonium p-toiuenesulfonate represented by Formula (1).

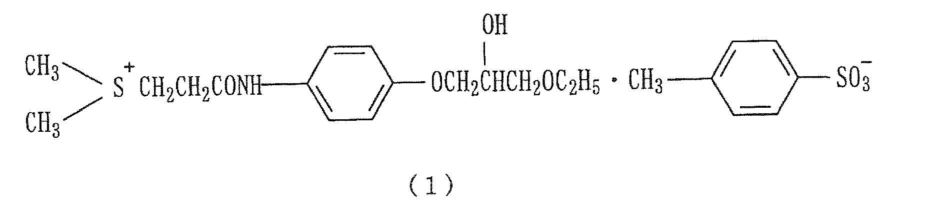

8. The method according to Claim 7, wherein the thrombosis is selected from the group consisting of myocardial infarction, brain infarction, pulmonary infarction, arteriosclerosis obliterans, Buerger disease, thrombophlebitis, venous thromboembolism (economy class syndrome), arterial thromboembolism, and disseminated intravascular coagulation (DIC).

9. (+)-[2-[4-(3-Ethoxy-2-hydroxypropoxy)phenylcarbamoyl]ethyl]dimethylsulfonium p-toluenesulfonate represented by Formula (1) for use in the treatment of thrombosis.

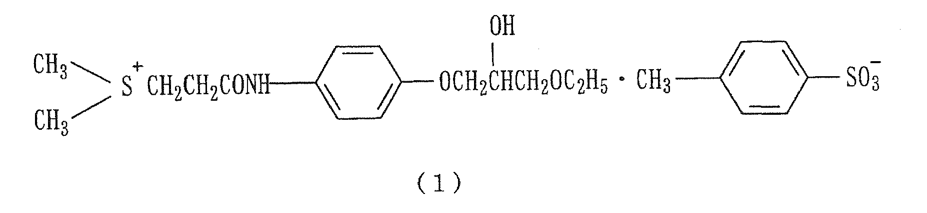

10. Use of (±)-[2-[4-(3-ethoxy-2-hydroxypropoxy)phenylcarbamoyl]ethyl]dimethylsulfonium p-toluenesulfonate represented by Formula (1) for the treatment of thrombosis.
